# EUROPÄISCHE PATENTANMELDUNG

(11) **EP 1 795 117 A1**
(43) Veröffentlichungstag der Anmeldung: **13.06.2007**
(21) Anmeldenummer: 05027064.4
(22) Anmeldetag: 12.12.2005
(51) Int. Cl.: A61B 5/00, A61M 5/00

(54) **Patientengerät mit abgesetzter Bedienoberfläche**

(71) Anmelder: F. HOFFMANN-LA ROCHE AG, 4070 Basel (CH); Roche Diagnostics GmbH, 68305 Mannheim (DE)
(72) Erfinder: Haueter, Ulrich, CH-3506 Grosshöchstetten (CH); Ducret, Maurice, CH-3072 Ostermundigen (CH); Bosshard, David, CH-3251 Wengi (CH); Rufer, Thomas, CH-3072 Ostermundigen (CH); Heutschi, Ivan, CH-3506 Grosshöchstetten (CH); Stoller, Hanspeter, CH-3012 Bern (CH); Jeckelmann, Joël, CH-1752 Villars-sur-Glâne (CH); Brügger, Martin, CH-3098 Köniz (CH); Kaufmann, Heiner, CH-3008 Bern (CH)
(74) Vertreter: Blum, Rudolf Emil

(57) **Zusammenfassung**

Ein Patientengerät (2) hat ein Gehäuse (3) und eine Bedienoberfläche für eine Interaktion zwischen einem Patienten und dem Patientengerät (2). Mindestens ein Teil der Bedienoberfläche ist vom Gehäuse (3) abgesetzt und für den Patienten erkennbar.

## Beschreibung

### Technisches Gebiet

Die Erfindung betrifft allgemein ein tragbares Patientengerät. Die Erfindung betrifft insbesondere die Bedienung des Patientengerätes.

### Stand der Technik

Tragbare Patientengeräte sind für eine Vielzahl von Anwendungen bekannt, beispielsweise zur Messung von physiologischen Parametern und zur Zufuhr von Medikamenten. Diese Patientengeräte sind mittlerweile wichtige Hilfsmittel bei der Diagnose und Therapie, die es den Patienten ermöglichen, dem gewohnten Alltag ohne grosse Einschränkungen nachzugehen, den Ärzten aber beispielsweise aussagekräftigere Messungen unter Alltagsbedingungen erlauben.

Ein beispielhaftes Patientengerät zur Messung eines physiologischen Parameters ist ein Blutzuckermessgerät, z. B. ein Accu-Chek® Blutzuckermesssystem der Roche Diagnostics GmbH, Deutschland. Das Blutzuckermessgerät analysiert auf einen Messstreifen aufgebrachtes Blut des Patienten und zeigt den gemessenen Blutzuckerwert, auch Glukosewert genannt, auf einer Anzeige an. Eine Bedienoberfläche für die Interaktion zwischen Patient und Gerät erlaubt es dem Patienten, gespeicherte Messwerte aufzurufen und auf der Anzeige darzustellen.

Ein beispielhaftes Patientengerät zur Medikamentzufuhr ist eine Insulinpumpe, z. B. eine Accu-Chek® Insulinpumpe der Roche Diagnostics GmbH, Deutschland. Ein Patient trägt die Insulinpumpe in der Regel am Körper. Über einen dünnen Schlauch, dessen Kanüle unter der Haut sitzt, gibt die Insulinpumpe kontinuierlich Insulin an den Körper ab. Mikroprozessoren steuern einen Motor, der z. B. alle drei Minuten über eine Gewindestange einen Stopfen in eine Insulinampulle bewegt. Diese Bewegung, die der jeweils programmierten Basalrate, d. h. des Insulingrundbedarfs des Patienten, entspricht, führt dem Körper die erforderliche Insulinmenge zu. Über eine Bedienoberfläche, einschliesslich einer Anzeigeeinheit (Display) und Tasten, kann der Patient die Insulinpumpe einstellen und bedienen, beispielsweise abhängig von einem vorgängig gemessenen Blutzuckerwert.

Ein Diabetespatient hat typischerweise immer ein Blutzuckermessgerät in greifbarer Nähe, beispielsweise als Set zusammen mit Messstreifen verpackt. Um dieses Set relativ bequem und diskret mit sich führen zu können, sollte das Patientengerät so klein wie möglich sein. Dies gilt auch für die Insulinpumpe, die der Patient möglichst bequem und diskret am Körper tragen möchte. Je mehr ein Patientengerät verkleinert wird, desto kleiner wird in der Regel die Bedienoberfläche. Darunter kann jedoch die Bedienerfreundlichkeit leiden.

### Darstellung der Erfindung

Es stellt sich daher die Aufgabe, ein Patientengerät zu schaffen, das trotz relativ kleinen Gehäuseabmessungen ein hohes Mass an Bedienerfreundlichkeit aufweist. Um dies zu ermöglichen, ist in den hier beschriebenen Ausführungsbeispielen eines Patientengerätes mindestens ein Teil der Bedienoberfläche des Patientengerätes, vor allem dessen Anzeigeeinheit, vom eigentlichen Patientengerät abgesetzt und für den Patienten erkennbar darstellbar.

Da mindestens ein Teil der Bedienoberfläche extern dargestellt wird, unterliegt die Grösse der Darstellung nicht mehr den Grössenbeschränkungen des Gehäuses des Patientengerätes. So kann die Bedienoberfläche und vor allem an den Patienten gerichtete Information grösser dargestellt werden als auf einem herkömmlichen Display eines Patientengerätes. Die Bedienoberfläche kann beispielsweise auf einem externen Display, einem Tisch oder einer Leinwand dargestellt werden, beispielsweise durch Projektion. Vor allem Diabetespatienten, die oft auch eine eingeschränkte Sehfähigkeit haben, können die grössere Darstellung besser erkennen.

In einem Ausführungsbeispiel ist ein Patientengerät, das eine Bedienoberfläche und ein Gehäuse aufweist, Teil eines Patientensystems. In diesem Patientensystem ist mindestens ein Teil der Bedienoberfläche vom Gehäuse abgesetzt und mittels einer Einrichtung darstellbar.

Je nach Ausführung kann die Bedienoberfläche ganz oder teilweise in dem Patientengerät integriert werden. Das evtl. unkomfortable Hantieren direkt am Patientengerät ist somit nicht nötig. Das Patientengerät kann dadurch auch kleiner sein, da je nach Ausführung nur noch eine minimale Bedienoberfläche, beispielsweise ein kleineres oder gar kein Display, im Patientengerät erforderlich ist.

### Kurze Beschreibung der Zeichnungen

Weitere Ausgestaltungen, Vorteile, neue Eigenschaften und Anwendungen der Erfindung ergeben sich aus der folgenden detaillierten Beschreibung unter Einbezug der Zeichnungen. In den Zeichnungen haben gleiche Elemente die gleichen Bezugszeichen. In den Zeichnungen zeigen:
Fig. 1 eine schematische Darstellung eines Ausführungsbeispiels eines Patientengerätes mit einer Projiziereinrichtung,
Fig. 2 und 3 schematische Darstellungen eines Ausführungsbeispiels eines Patientengerätes mit einem Display und einer Projiziereinrichtung,
Fig. 4 eine schematische Darstellung eines Ausführungsbeispiels eines Systems mit einem Patientengerät und einer externen Bedienoberfläche, und
Fig. 5 eine schematische Darstellung eines Ausführungsbeispiels eines Patientengerätes mit vergrösserter Bedienoberfläche.

### Wege zur Ausführung der Erfindung

Die verschiedenen Wege zur Ausführung der Erfindung sind im Folgenden ohne Einschränkung des Schutzbereiches mit Bezug auf Geräte für die Behandlung von Diabetes, beispielsweise eine Insulinpumpe oder ein Blutzuckermessgerät, beschrieben. Diese Patientengeräte sind jedoch nur zwei Beispiele für Patientengeräte, die möglichst klein sein und trotzdem ein hohes Mass an Bedienerfreundlichkeit haben sollen. Pumpen für andere Medikamente, Laktatmessgeräte, Puls- und Blutdruckmessgeräte und EKG Geräte sind weitere Beispiele für Patientengeräte, bei denen die Erfindung Anwendung finden kann.

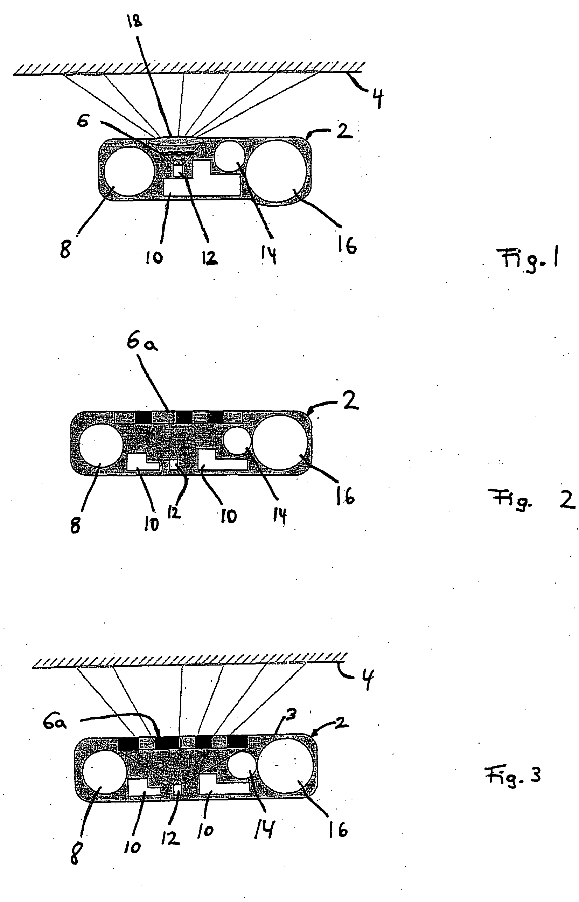

Figur 1 zeigt eine schematische Darstellung eines Ausführungsbeispiels eines Patientengerätes 2, beispielsweise einer Insulinpumpe. Das Patientengerät 2 hat ein Gehäuse 3 und eine Projiziereinrichtung, die Information auf einer vom Patientengerät 2 entfernten Oberfläche 4 darstellt. Die Oberfläche 4 kann beispielsweise ein Tisch, eine Wand, eine Leinwand, ein Blatt Papier, ein Kleidungsstück oder ein Handrücken sein. Die projizierte Information kann beispielsweise die eingestellte Basalrate beinhalten. In anderen Ausführungsbeispielen kann die projizierte Information zusätzliche Information beinhalten, beispielsweise die Einheit der Basalrate, das Datum und die Uhrzeit. Im Allgemeinen ist unter dem Begriff Information jegliche Art von Daten, die für den Betrieb des Gerätes und für den Patienten relevant sind, zu verstehen und zwar unabhängig von der Art ihrer Darstellung, beispielsweise als alphanumerischer Text, Symbol, Graphik, Bild, Video oder optisches, evtl. farblich codiertes Signal.

Die Grösse der projizierten Information kann auf die Sehfähigkeit des Patienten angepasst sein. Je nach Bedarf und abhängig von der Distanz zwischen Patientengerät 2 und Oberfläche 4 kann die gewünschte Grösse eingestellt werden. Vor allem bei einer eingeschränkten Sehfähigkeit des Patienten (z. B. durch Diabetes verursacht) kann die projizierte Information grösser als auf einem herkömmlichen, im Gerät integrierten Display dargestellt werden. Die Lesbarkeit der Information wird dadurch wesentlich erhöht. Bei (noch) guter Sehfähigkeit kann die projizierte Information relativ klein dargestellt werden, wodurch eine diskrete Handhabung des Patientengerätes 2 möglich ist.

Im gezeigten Ausführungsbeispiel hat die Projiziereinrichtung einen Projektor, der durch eine Lichtquelle 12 für sichtbares Licht (z. B. eine Leuchtdiode (LED) oder ein Laser), eine optische Linse 18 und eine zwischen der Lichtquelle 12 und der Linse 18 angeordnete Abbildungsmatrix 6 (z. B. ein LCD Panel) angedeutet ist. Eine Steuerelektronik 10 ist an die Projiziereinrichtung gekoppelt und generiert Steuersignale zur Ansteuerung der Abbildungsmatrix 6. Bei einem LCD Panel als Abbildungsmatrix 6 werden durch die Steuersignale einzelne Pixel angesteuert, die daraufhin entweder Licht durchlassen oder sperren, um so ein Bild auf die Oberfläche 4 zu projizieren.

Die prinzipielle Funktionsweise von Projektoren ist allgemein bekannt. Relativ kleine, monochrome Projektoren sind beispielsweise aus der Verwendung in elektronischen Weckern bekannt, die die Uhrzeit auf eine Wand projizieren. In einem Ausführungsbeispiel kann das Patientengerät 2 einen aus der Weckeranwendung bekannten Projektor aufweisen. Alternativ zu diesem Projektor kann in einem anderen Ausführungsbeispiel ein vom Fraunhofer Institut für Siliziumtechnologie entwickelter Miniaturbeamer, oder ein Field Lens Design^{™} Projektor der Firma Carl Zeiss AG verwendet werden.

Das Patientengerät 2 hat ausserdem eine Antriebseinrichtung 14 (z. B. einen Motor und ein Getriebe), die auf eine Insulinampulle 16 wirkt und Insulin aus der Ampulle unter die Haut des Patienten pumpt. Eine Spannungsquelle 8, beispielsweise eine Batterie, liefert Energie für den Betrieb des Patientengerätes 2.

Die Projiziereinrichtung ist Teil einer Bedienoberfläche (oft auch als Userinterface bezeichnet) des Patientengerätes 2, über die die Interaktion zwischen Patient und Gerät erfolgt, beispielsweise die Bedienung des Patientengerätes 2 durch den Patienten. Zur Bedienoberfläche gehören typischerweise Ein- und Ausgabeeinrichtungen, beispielsweise Anzeigeeinheiten (Displays) und Bedientasten (evtl. eine Tastatur oder ein sogenannter Touchscreen). Es versteht sich daher, dass das Patientengerät 2 ausser den genannten Komponenten weitere, nicht gezeigte Komponenten hat, beispielsweise Bedientasten, mittels derer der Patient das Patientengerät 2 bedienen kann.

In dem gezeigten Ausführungsbeispiel hat das Patientengerät 2 keine Anzeigeeinheit. Die für den Patienten oder Dritte darzustellende Information wird auf die Oberfläche 4 projiziert. Das Gehäuse 3 des Patientengerätes 2 kann daher kleiner dimensioniert werden.

Figuren 2 und 3 zeigen schematische Darstellungen eines Ausführungsbeispiels eines Patientengerätes 2 mit einer Anzeigeeinheit 6a, im Folgenden auch Display 6a genannt, und einer Projiziereinrichtung. Ähnlich der in Figur 1 gezeigten Ausführung kann das Display 6a ein LCD Panel sein, das durch die Lichtquelle 12 für sichtbares Licht beleuchtet wird. Die Steuerelektronik 10 ist an die Projiziereinrichtung gekoppelt und generiert Steuersignale zur Ansteuerung des Displays 6a.

Das Display 6a hat eine Doppelfunktion, d. h. es dient als Anzeigeeinheit und als Abbildungs- oder Projektionsmatrix. In Figur 2 ist die Lichtquelle 12 nicht aktiviert, so dass das Display 6a nicht von hinten beleuchtet ist. Das Display 6a stellt in dieser Anwendung und gesteuert von der Steuerelektronik 10 die Information dar. Der Patient kann damit die Information direkt vom Display 6a ablesen. In Figur 3 beleuchtet die Lichtquelle 12 dagegen das Display 6a, das als Abbildungsmatrix wirkt, wodurch die Information auf die Oberfläche 4 projiziert wird. Die Oberfläche 4 kann ein Tisch oder eine der oben genannten Oberflächen sein.

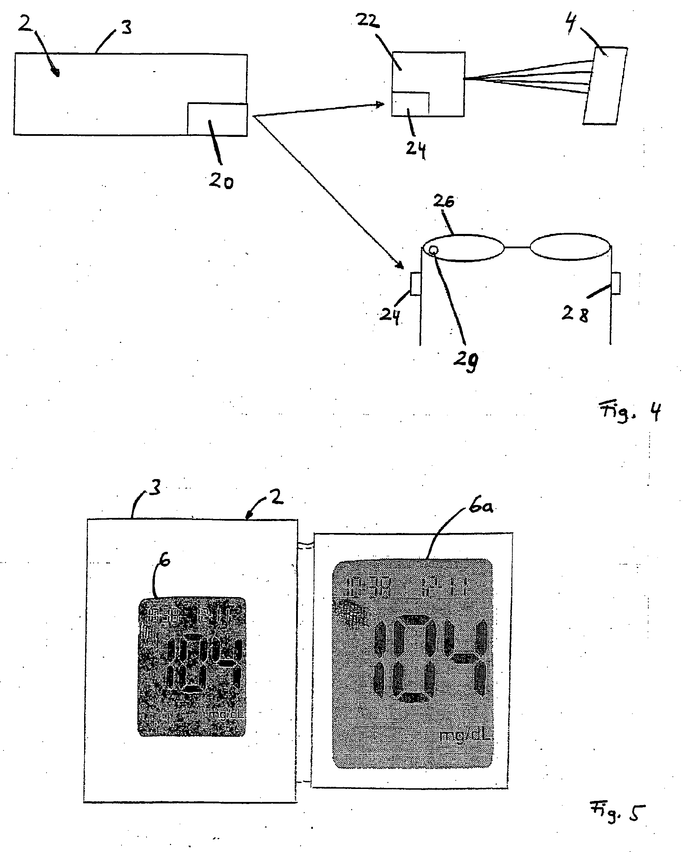

Figur 4 zeigt eine schematische Darstellung eines Ausführungsbeispiels eines Systems mit einem Patientengerät 2 und einer externen Bedienoberfläche. Das Patientengerät 2, beispielsweise eine Insulinpumpe, hat neben den genannten Komponenten wie Antriebseinrichtung, Insulinampulle und Spannungsquelle, einen Sender 20. Im gezeigten System kann der Sender 20 Daten drahtlos oder drahtgebunden an eine Projiziereinrichtung 22 oder drahtlos an ein vom Patienten getragenes Hilfsmittel 26, beispielsweise eine Brille 26, senden. Die Projiziereinrichtung 22 und die Brille 26 haben dazu jeweils einen Empfänger 24.

In einem Ausführungsbeispiel kann der Sender 20 Daten drahtlos gemäss dem Bluetooth® Standart (Serial Port Profile (SSP)) mit einem darüber liegenden Protokoll der Applikationsebene senden. In einem anderen Ausführungsbeispiel kann der Sender 20 die Daten gemäss dem Ir-DA, WLAN (802.11x), Zigbee, WiMax oder Mics Protokoll senden.

Das Hilfsmittel 26, bzw. die Brille 26, hat ausserdem eine Spannungsquelle 28 und eine Lichtquelle 29, beispielsweise eine ein- oder mehrfarbige Leuchtdiode, die so an der Brille 26 befestigt ist, dass der Patient von ihr emittiertes Licht sehen kann. Dem Patienten kann mittels der Lichtquelle 29 Information über den Zustand des Patientengerätes 2 übermittelt werden, beispielsweise eine Warnmeldung wenn es sich im Stoppmodus befindet, Fehler- oder Alarmmeldungen, eine Bestätigung nach Betätigen einer Eingabetaste, oder eine Bestätigung der eingestellten Bolusmenge durch mehrmalige optische Impulse.

In einem Ausführungsbeispiel ersetzen die optischen Impulse die üblicherweise in Patientengeräten verwendeten akustischen Signale, die unter Umständen vom Patienten oder seiner Umgebung als störend empfunden werden können. In einem anderen Ausführungsbeispiel können die akustischen Signal zusätzlich zu den optischen Impulsen verwendet werden. Das Patientengerät 2 kann dabei je nach Patient und Umgebung bei Bedarf die akustischen Signal unterdrücken.

Die Projiziereinrichtung 22 kann die genannte Information über den Zustand des Patientengerätes 2 auf die Oberfläche 4 projizieren. Darüber hinaus kann weitere Information als Text, Symbole, Graphik, Bild oder Video auf die Oberfläche 4 projiziert werden.

In einem anderen Ausführungsbeispiel können die mit Bezug auf die Figuren 1 - 4 beschriebenen Projiziereinrichtungen so ausgestaltet sein, dass sie auf der Oberfläche 4 eine virtuelle Tastatur darstellen können. Eine Einrichtung zur Darstellung einer virtuellen Tastatur ist von VKB Inc., USA, kommerziell erhältlich. Die Einrichtung besteht aus einem Projektionsmodul (mit einer roten Laserdiode und einer Optik für die Projektion), einem Beleuchtungsmodul (infraroten Laserdiode mit Optik) und einem Detektionsmodul (Infrarotkamera und Auswerteelektronik). Wenn ein Buchstabe auf der projizierten Tastatur angetippt wird, reflektiert der Finger infrarotes Licht, das die Kamera detektiert. Die Auswerteelektronik ermittelt daraus den Buchstaben und überträgt ihn auf das Display.

Diese Einrichtung kann als Projiziereinrichtung im Patientengerät 2 integriert sein, wie beispielsweise in Figur 1 angedeutet. Alternativ kann die Einrichtung als Projiziereinrichtung an das Patientengerät 2 gekoppelt sein, wie beispielsweise in Figur 4 angedeutet. Die Kopplung kann mittels einer drahtlosen oder drahtgebundenen Übertragungstrecke erfolgen.

Es versteht sich, dass die Projiziereinrichtungen so ausgestaltet sein können, dass sie nicht nur die darzustellende Information auf die Oberfläche 1 projizieren können, sondern auch die virtuelle Tastatur. Die virtuelle Tastatur erlaubt es dem Patienten, Therapiedaten schnell und einfach einzugeben. Dabei kann der Patient die eingegebenen Daten bequem auf der projizieren Darstellung überprüfen.

Figur 5 zeigt eine schematische Darstellung eines Ausführungsbeispiels eines Patientengerätes 2 mit externer und vergrösserter Bedienoberfläche. Im gezeigten Ausführungsbeispiel hat das Patientengerät 2 ein Display 6 und ein externes, an das Patientengerät 2 gekoppeltes Display 6a. Wie in Figur 5 dargestellt, ist das externe Display 6a grösser als das Display 6. Die darzustellende Information kann damit auf dem externen Display 6a grösser und somit besser lesbar dargestellt werden. In einem weiteren Ausführungsbeispiel ist kein Display 6 im Patientengerät 2 vorgesehen, sondern nur das externe Display 6a.

Das externe Display 6a kann ein konventionelles LCD Display sein, das klappbar oder schwenkbar am Patientengerät 2 befestigt ist. Dazu kann das Display 6a mittels eines Gelenks oder Scharniers am Patientengerät 2 befestigt sein. Über das Gelenk oder Scharnier erfolgt auch der elektrische Anschluss des externen Displays 6a. Derartige Gelenke oder Scharniere werden beispielsweise in klappbaren Mobilfunktelefonen oder Laptop-Computern verwendet.

In einem anderen Ausführungsbeispiel kann das Patientengerät 2 einen Schacht haben, aus dem das externe Display 6a bei Bedarf herausgezogen werden kann. Zum elektrischen Anschluss des externen Displays 6a können ein flexibles Flachbandkabel oder Schleifkontakte verwendet werden.

Alternativ zu einem starren LCD Display kann ein flexibles Display verwendet werden, das bei Bedarf ausgerollt werden kann. Ein flexibles Display kann aus organischen lichtemittierenden Dioden (OLED) aufgebaut sein. Ein solches Display ist beispielsweise vom Fraunhofer-Institut für Angewandte Polymerforschung entwickelt worden.

Die genannten Ausführungsbeispiele erlauben die Darstellung von Information auf einem grösseren Display 6a. Je nach Ausführung kann das Display 6a nahezu die Grösse des Patientengerätes 2 haben. Dadurch ist die Information vor allem von Patienten mit eingeschränktem Sehvermögen besser lesbar. Ausserdem kann das Display 6a bei Nichtgebrauch platzsparend verbaut werden.

Die im vorhergehenden beschriebenen Ausführungsbeispiele eines Patientengerätes oder Patientensystems ermöglichen, dass mindestens ein Teil der Bedienoberfläche des Patientengerätes 2 vom Gehäuse 3 abgesetzt darstellbar ist. Je nach gewünschter Anwendung kann bei den gezeigten Ausführungsbeispielen die Anzeigeinheit entfallen. Dadurch kann das Patientengerät 2 bei Bedarf kleiner konfiguriert werden, ohne dass dabei die Bedienerfreundlichkeit leidet.

## Patentansprüche

1. Patientengerät (2) mit einem Gehäuse (3) und einer Bedienoberfläche für eine Interaktion zwischen einem Patienten und dem Patientengerät (2), **dadurch gekennzeichnet, dass** mindestens ein Teil der Bedienoberfläche vom Gehäuse (3) abgesetzt darstellbar und für den Patienten erkennbar ist.

2. Patientengerät (2) nach Anspruch 1, bei dem das Gehäuse (3) eine Projiziereinheit hat, die Information auf eine vom Gehäuse (3) abgesetzte Oberfläche (4) projiziert, um die Information dem Patienten anzuzeigen.

3. Patientengerät (2) nach Anspruch 2, bei dem die Projiziereinheit eine optische Linse (18), eine Lichtquelle (12) für sichtbares Licht und eine zwischen der Linse (18) und der Lichtquelle (12) angeordnete Abbildungsmatrix (6) hat, wobei die darzustellende Information von der Linse (18) auf die Oberfläche (4) projiziert wird.

4. Patientengerät (2) nach Anspruch 2, bei dem die Projiziereinheit eine Lichtquelle (12) für sichtbares Licht und eine in einer Gehäusewand positionierte Abbildungsmatrix (6a) hat, wobei die Abbildungsmatrix (6a) die darzustellende Information bei eingeschalteter Lichtquelle (12) auf die Oberfläche (4) projiziert, und wobei die Information bei ausgeschalteter Lichtquelle (12) auf der Abbildungsmatrix (6a) dargestellt ist.

5. Patientengerät (2) nach einem der Ansprüche 2 bis 4, bei dem die Projiziereinheit eine virtuelle Tastatur auf der Oberfläche (4) darstellt, die die Eingabe von Daten durch den Patienten ermöglicht.

6. Patientengerät (2) nach Anspruch 1, bei dem eine externe Anzeigeeinheit (6a) an das Gehäuse (3) gekoppelt ist, auf der die Information darstellbar ist.

7. Patientengerät (2) nach Anspruch 6, bei dem die externe Anzeigeeinheit (6a) klappbar, schwenkbar, ausziehbar oder ausrollbar an das Gehäuse (3) gekoppelt ist.

8. Patientengerät (2) nach einem der Ansprüche 6 bis 7, bei dem das Gehäuse (3) eine Anzeigeeinheit (6) hat, auf der die Information darstellbar ist.

9. Patientensystem mit einem Patientengerät (2), das eine Bedienoberfläche und ein Gehäuse (3) aufweist, **dadurch gekennzeichnet, dass** mindestens ein Teil der Bedienoberfläche vom Gehäuse (3) abgesetzt mittels einer Einrichtung (22, 26) darstellbar und für den Patienten erkennbar ist.

10. Patientensystem nach Anspruch 9, bei dem das Patientengerät (2) einen Sender (20) hat und die Einrichtung (22, 26) einen zum Sender (20) kompatiblen Empfänger (24) hat, wobei der Sender (20) Daten zum Empfänger (24) sendet, um mindestens einen Teil der Bedienoberfläche darzustellen.

11. Patientensystem nach Anspruch 10, bei dem die Einrichtung (22) eine Projiziereinrichtung ist, die mindestens einen Teil der Bedienoberfläche auf einer Oberfläche (4) darstellt.

12. Patientensystem nach Anspruch 10, bei dem die Einrichtung (26) ein vom Patienten tragbares Hilfsmittel ist, das eine Lichtquelle (29) hat, um die Daten als optische Signale darzustellen.
